# NEW EUROPEAN PATENT SPECIFICATION

(11) **EP 1 701 668 B2**
(45) Date of publication and mention of the opposition decision: **28.08.2019**
(45) Mention of the grant of the patent: 27.03.2013
(21) Application number: 04813777.2
(22) Date of filing: 10.12.2004
(51) Int. Cl.: A61F 2/24, A61F 2/01

(54) **APPARATUS FOR HEART VALVE REPLACEMENT**
GERÄT FÜR DEN HERZKLAPPENERSATZ
APPAREILS DU REMPLACEMENT ENDOVASCULAIRE DE LA VALVULE CARDIAQUE

(30) Priority: 23.12.2003 US 746280; 17.08.2004 US 920736
(43) Date of publication of application: 20.09.2006
(73) Proprietor: Boston Scientific Corporation, Maple Grove, Minnesota 55311 (US)
(72) Inventor: SALAHIEH, Amr, Saratoga, CA 95070 (US); BRANDT, Brian, Santa Clara, CA 95051 (US); MOREJOHN, Dwight, P., Davis, CA 95616 (US); MICHLITSCH, Kenneth, J., Livermore, CA 94550 (US); SAUL, Tom, El Granada, CA 94018 (US)
(74) Representative: Vossius & Partner Patentanwälte Rechtsanwälte mbB
(86) International application number: PCT/US2004/041513
(87) International publication number: WO 2005/065585

(56) References cited:
- WO-A1-00/21604
- WO-A1-00/44313
- WO-A1-01/35870
- WO-A1-2004/006803
- WO-A1-2004/006804
- WO-A1-2004/019817
- WO-A2-03/105721
- WO-A2-2004/021922
- US-A- 5 411 552
- US-A- 5 667 523
- US-A- 5 957 949
- US-A- 5 957 949
- US-A1- 2003 040 736
- US-A1- 2005 137 688
- US-A1- 2005 157 896
- Cerebral Protection with Filter Devices during Carotid Artery Stenting
- Particulate Emboli Capture by an Intra-Aortic Filter Device during Cardiac Surgery

## Description

### BACKGROUND OF THE INVENTION

The present invention relates to methods and apparatus for protecting a patient from embolization during endovascular replacement of the patient's heart valve. More particularly, the present invention relates to methods and apparatus for providing embolic protection by filtering blood downstream of the valve during endovascular replacement.

Heart valve surgery is used to repair or replace diseased heart valves. Valve surgery typically is an open-heart procedure conducted under general anesthesia. An incision is made through a patient's sternum (sternotomy), and the patient's heart is stopped while blood flow is rerouted through a heart-lung bypass machine. The valve then is surgically repaired or replaced, blood is rerouted back through the patient's heart, the heart is restarted, and the patient is sewn up.

Valve replacement may be indicated when there is a narrowing of the native heart valve, commonly referred to as stenosis, or when the native valve leaks or regurgitates. When replacing the valve, the native valve is excised and replaced with either a biologic or a mechanical valve. Mechanical valves require lifelong anticoagulant medication to prevent blood clot formation, and clicking of the valve often may be heard through the chest. Biologic tissue valves typically do not require such medication. Tissue valves may be obtained from cadavers or may be porcine or bovine, and are commonly attached to synthetic rings that are secured to the patient's heart.

Valve replacement surgery is a highly invasive operation with significant concomitant risk. Risks include bleeding, infection, stroke, heart attack, arrhythmia, renal failure, adverse reactions to the anesthesia medications, as well as sudden death. 2-5% of patients die during surgery.

Post-surgery, patients temporarily may be confused due to emboli and other factors associated with the heart-lung machine. The first 2-3 days following surgery are spent in an intensive care unit where heart functions can be closely monitored. The average hospital stay is between 1 to 2 weeks, with several more weeks to months required for complete recovery.

In recent years, advancements in minimally invasive surgery and interventional cardiology have encouraged some investigators to pursue percutaneous, endovascular replacement of the aortic heart valve. See, e.g., U.S. Pat. No. 6,168,614. The replacement valve may be deployed across the native diseased valve to permanently hold the native valve open, thereby alleviating a need to excise the native valve and to position the replacement valve in place of the native valve. Optionally, a valvuloplasty may be performed prior to, or after, deployment of the replacement valve. US 2003/040736 discloses venting catheters. The system can comprise a blood filter unit outside the body.

Since the native valve may be calcified or stenosed, valvuloplasty and/or deployment of the replacement valve poses a risk of loosening and releasing embolic material into the patient's blood stream. This material may, for example, travel downstream through the patient's aorta and carotids to the cerebral vasculature of the brain. Thus, a risk exists of reduction in mental faculties, stroke or even death during endovascular heart valve replacement, due to release of embolic material.

In view of the foregoing, it would be desirable to provide methods and apparatus for protecting against embolization during endovascular replacement of a patient's heart valve.

### SUMMARY OF THE INVENTION

One aspect of the invention provides apparatus for protecting against embolization during endovascularly replacement of a patient's heart valve, including: a replacement valve configured for endovascular delivery and deployment; and an embolic filter configured for disposal downstream of the replacement valve during endovascular deployment of the valve.

An example provides a method for protecting a patient against embolization during endovascular replacement of the patient's heart valve, including the steps of: endovascularly delivering a replacement valve to a vicinity of the patient's heart valve; endovascularly deploying an embolic filter downstream of the heart valve; and endovascularly deploying the replacement valve. The method may also include the step removing the embolic filter from the patient after endovascular deployment of the replacement valve. In embodiments in which the heart valve is an aortic valve, the endovascular delivery step may include the step of endovasculary delivering the replacement valve along a retrograde approach, and the filter deployment step may include deploying the filter in the patient's aorta. The method may also include the step of endovascularly delivering an expandable balloon to the vicinity of the heart valve and performing valvuloplasty with the expandable balloon.

Yet another aspect of the invention provides apparatus for protecting against embolization during endovascularly replacement of a patient's heart valve, including: a delivery catheter having an expandable replacement valve disposed therein; and an embolic filter advanceable along the delivery catheter for diverting emboli released during endovascular deployment of the replacement valve. The invention is disclosed in the appended claims.

### INCORPORATION BY REFERENCE

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figures 1A-F are side views, partially in section, illustrating a method and apparatus for protecting a patient against embolization during endovascular replacement of the patient's diseased aortic valve.
Figure 2 is a side view, partially in section, illustrating an alternative embodiment of the apparatus and method of Figures 1.
Figures 3A-D are schematic side-sectional views illustrating another alternative method and apparatus for protecting against embolization during endovascular valve replacement.
Figures 4A-D are side-views, partially in section, illustrating yet another method and apparatus for protecting against embolization, wherein an embolic filter is coaxially advanced over, or coupled to, an exterior of a replacement valve delivery catheter.
Figures 5A-F are schematic isometric views illustrating alternative embodiments of the apparatus of Figures 4.
Figures 6A-D are side views, partially in section, illustrating another method and apparatus for protecting against embolization.
Figure 7A-B are cross- and side-sectional detail views, respectively, along section lines A--A and B--B of Figure 6A, respectively, illustrating an optional method and apparatus for enhancing blood flow to the patient's coronary arteries while utilizing the apparatus of Figures 6.
Figure 8 is a schematic view of an embodiment of the apparatus of Figures 6 comprising a measuring element.
Figures 9A-I are schematic views of exemplary alternative embodiments of the apparatus of Figures 6.
Figures 10A-B are detail schematic views illustrating a spiral wound support structure.
Figure 11 is a detail schematic view illustrating longitudinal supports for maintaining a length of the apparatus.
Figures 12A-C are detail schematic views illustrating alternative deployment and retrieval methods for the apparatus.
Figures 13A-G are schematic views and side views, partially in section, illustrating a method and apparatus for protecting a patient against embolization during endovascular valvuloplasty and replacement of the patient's diseased aortic valve.

### DETAILED DESCRIPTION OF THE INVENTION

While preferred embodiments of the present invention are shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

The present invention relates to methods and apparatus for protecting a patient against embolization during endovascular replacement of the patient's diseased heart valve. More particularly, the present invention relates to methods and apparatus for providing embolic protection by filtering blood downstream of the valve during endovascular replacement. Applicant has previously described methods and apparatus for endovascularly replacing a patient's diseased heart valve, for example, in co-pending United States Patent Application Serial No. 10/746,280, filed December 23, 2003, from which the present application claims priority.

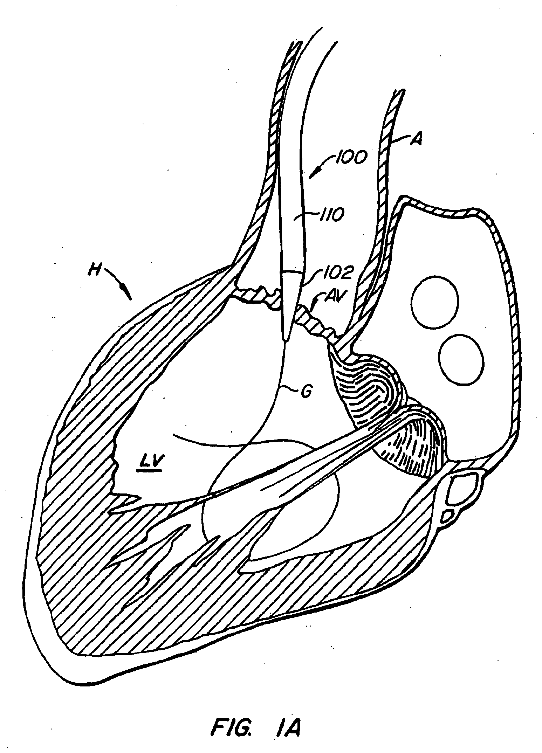

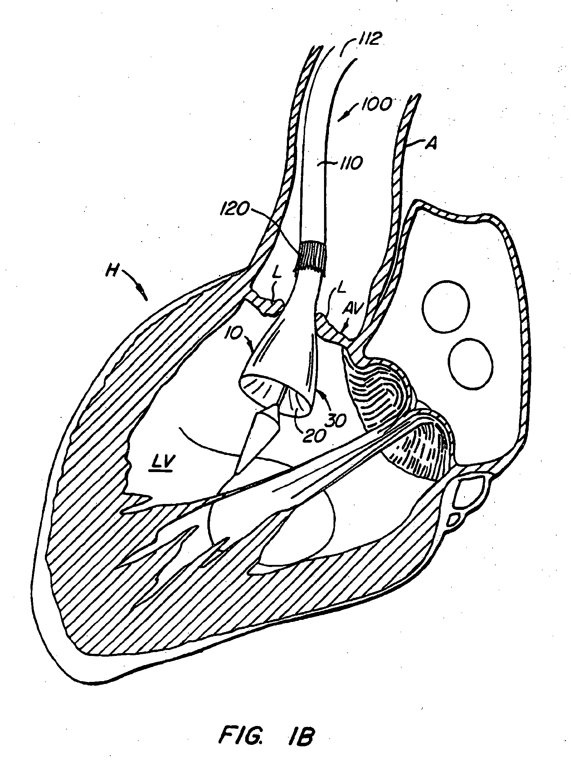

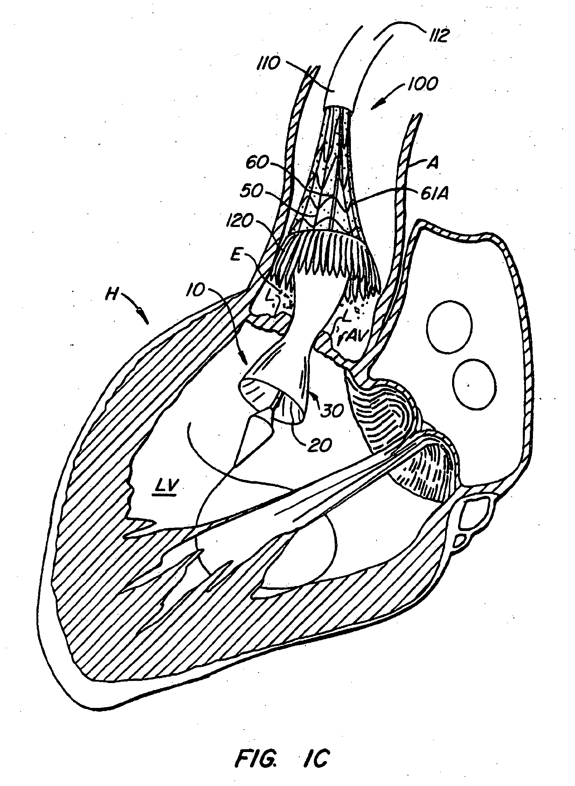

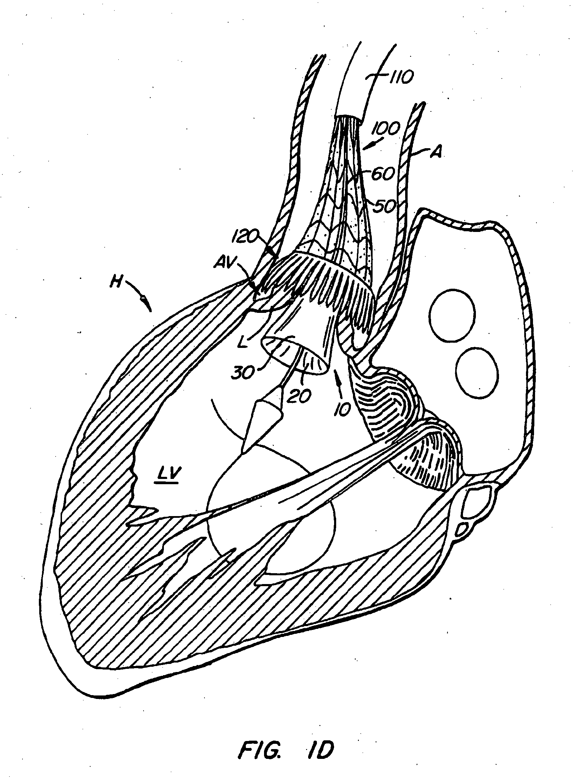

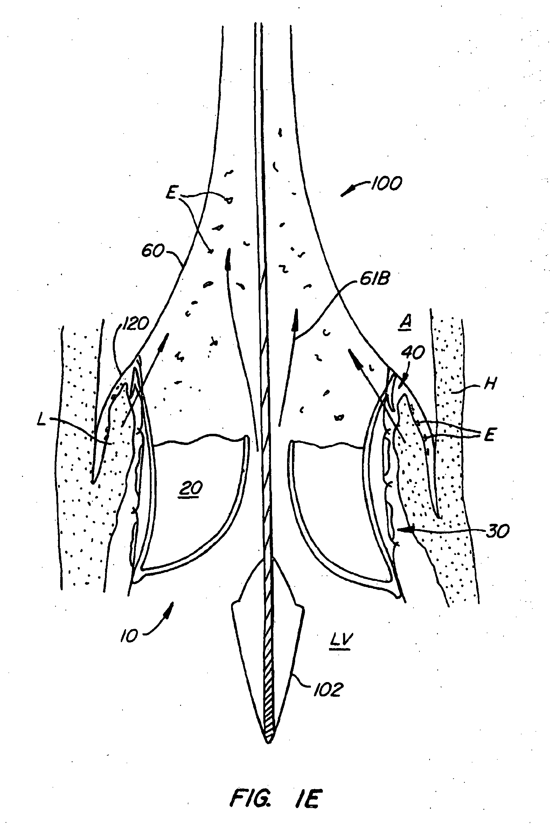

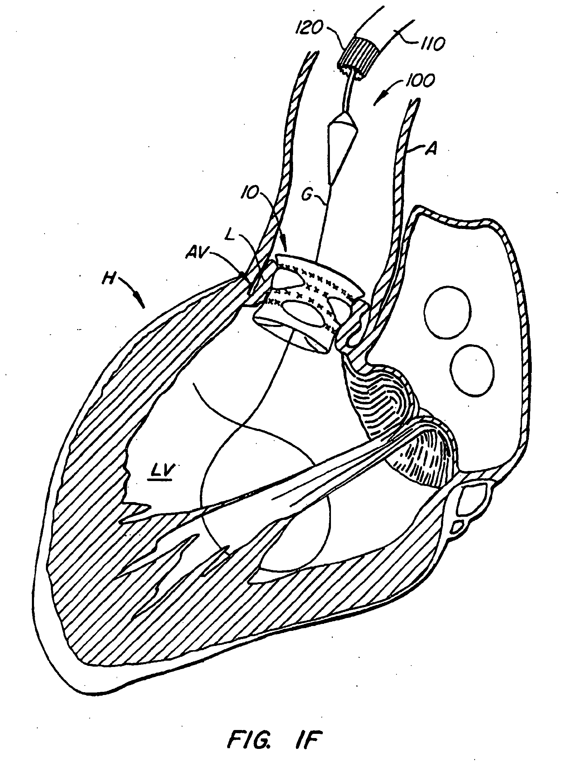

Referring now to Figures 1, a first embodiment of a method and apparatus for protecting a patient against embolization during endovascular replacement of the patient's diseased aortic valve is described. In Figures 1, replacement valve apparatus 10 illustratively comprises replacement valve 20 disposed within and coupled to expandable anchor 30. Apparatus 10 is provided only for the sake of illustration, and any other replacement valve apparatus may alternatively be provided.

Replacement valve 20 preferably is from biologic tissues, e.g. porcine valve leaflets or bovine or equine pericardium tissues. Alternatively, it can be made from tissue-engineered materials (such as extracellular matrix material from Small Intestinal Submucosa (SIS)). As yet another alternative, the replacement valve may be prosthetic from an elastomeric polymer or silicone, or a Nitinol or stainless steel mesh or pattern (sputtered, chemically milled or laser cut). Replacement valve 20 may comprise leaflets that may also be made of a composite of the elastomeric or silicone materials and metal alloys or other fibers, such Kevlar or carbon. Anchor 30 may, for example, dynamically self-expand; expand via a hydraulic or pneumatic force, such as expansion of a balloon catheter therein; expand via a non-hydraulic or non-pneumatic force; and/or be foreshortened in order to increase its radial strength.

Replacement valve apparatus 10 is reversibly coupled to delivery system 100, which illustratively comprises sheath 110 having lumen 112, as well as control wires 50 and control rods or tubes 60. Delivery system 100 may further comprise leaflet engagement element 120, as well as filter structure 61A. Engagement element 120, which may be releasably coupled to the anchor, is disposed between the anchor and tubes 60 of the delivery system. Filter structure 61 A may, for example, comprise a membrane or braid, e.g., an expandable Nitinol braid, circumferentially disposed about tubes 60. Structure 61A preferably comprises a specified porosity, for example, preferably comprises a plurality of pores on the order of about 100 µm or less to facilitate blood flow therethrough while filtering dangerously sized emboli from the blood. Structure 61A may be used independently or in combination with engagement element 120 to provide embolic protection during deployment of replacement valve apparatus 10.

Replacement valve apparatus 10 is configured for disposal in a delivery configuration within lumen 112 of sheath 110 to facilitate percutaneous, endoluminal delivery thereof. Wires 50, tubes 60, element 120 and/or sheath 110 of delivery system 100 may be utilized to deploy apparatus 10 from the delivery configuration to an expanded deployed configuration.

In Figure 1A, sheath 110 of delivery system 100, having apparatus 10 disposed therein, may be endovascularly advanced over guide wire G, preferably in a retrograde fashion (although an antegrade or hybrid approach alternatively may be used), through a patient's aorta **A** to the patient's diseased aortic valve **AV**. A nosecone 102 precedes sheath 110 in a known manner. In Figure 1B, sheath 110 is positioned such that its distal region is disposed within left ventricle **LV** of the patient's heart **H**.

After properly aligning the apparatus relative to anatomical landmarks, such as the patient's coronary ostia or the patient's native valve leaflets **L**, apparatus 10 may be deployed from lumen 112 of sheath 110, for example, under fluoroscopic guidance. Anchor 30 of apparatus 10 illustratively self-expands to a partially deployed configuration, as in Figure 1C. Leaflet engagement element 120 of delivery system 100 preferably self-expands along with anchor 30.

Element 120 initially is deployed proximal of the patient's native valve leaflets **L**, such that the element sealingly engages against the patient's aorta A to capture or otherwise filter emboli **E** that may be released during maneuvering or deployment of apparatus 10. Element 120 may also direct emboli **E** into filter structure 61 A and out through sheath 110, such that the emboli do not travel downstream through the patient's aorta or into the patient's cerebral vasculature. Suction optionally may be drawn through lumen 112 of sheath 110 during placement of apparatus 10 to facilitate aspiration or removal of emboli **E** from the patient's blood stream to further reduce a risk of embolization.

As seen in Figure 1D, apparatus 10 and element 120 may be advanced, and/or anchor 30 may be foreshortened, until the engagement element positively registers against valve leaflets **L**, thereby ensuring proper positioning of apparatus 10. Upon positive registration of element 120 against leaflets **L**, element 120 precludes further distal migration of apparatus 10 during additional foreshortening or other deployment of apparatus 10, thereby reducing a risk of improperly positioning the apparatus. Once expanded to the fully deployed configuration of Figure 1D, replacement valve apparatus 10 regulates normal blood flow between left ventricle **LV** and aorta **A**.

As discussed, emboli can be generated during manipulation and placement of apparatus 10, e.g., from the diseased native leaflets or from surrounding aortic tissue. Arrows 61B in Figure 1E show blood flowing past engagement element 120 and through porous filter structure 61A. While blood is able to flow through the filter structure, emboli **E** are trapped in the delivery system and removed with it at the end of the procedure or aspirated via suction during the procedure. Figure 1E also details engagement of element 120 against the native leaflets and illustrates locks 40, which optionally may be used to maintain apparatus 10 in the fully deployed configuration.

As seen in Figure 1F, delivery system 100 may be decoupled from apparatus 10 and removed from the patient, thereby removing the embolic filter provided by element 120 and filter structure 61 A, and completing protected, beating-heart, endovascular replacement of the patient's diseased aortic valve.

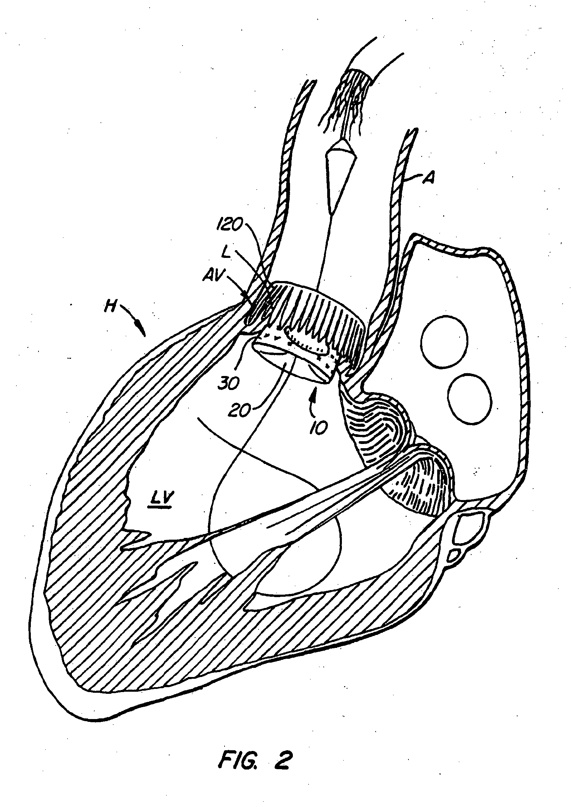

With reference to Figure 2, an alternative embodiment of the apparatus of Figures 1 is described, wherein leaflet engagement element 120 is coupled to anchor 30 of apparatus 10, rather than to delivery system 100. Engagement element 120 remains implanted in the patient post-deployment of apparatus 10, and leaflets **L** of native aortic valve **AV** are sandwiched between the engagement element and anchor 30. In this manner, element 120 positively registers apparatus 10 relative to the leaflets and precludes distal migration of the apparatus over time. Furthermore, since element 120 may act as an embolic filter during deployment of apparatus 10, any emboli **E** captured against element 120 may harmlessly remain sandwiched between the element and the patient's native leaflets, thereby reducing a risk of embolization.

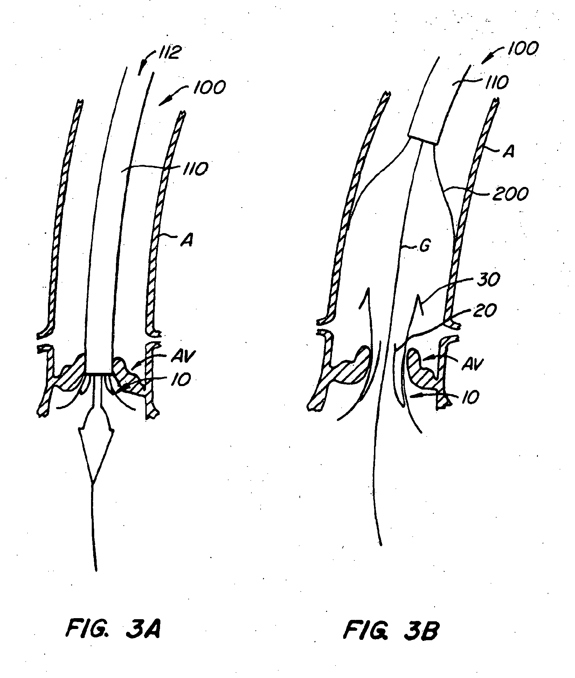

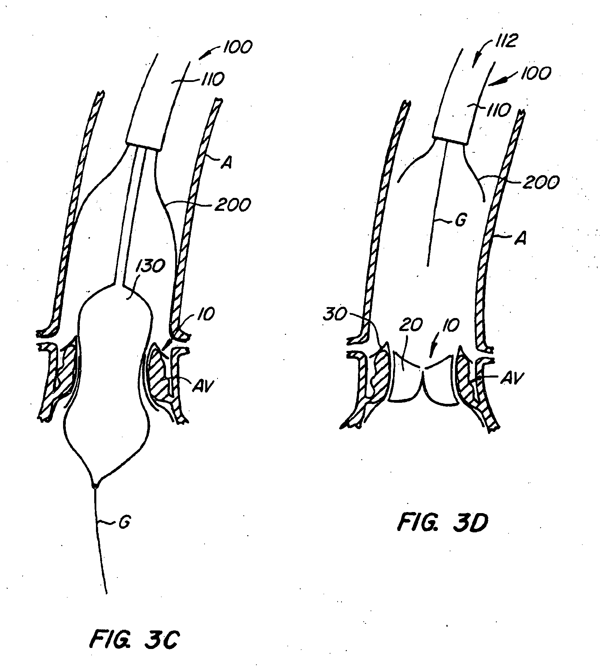

Referring now to Figures 3, another alternative method and apparatus for protecting against embolization is described. In Figure 3A, replacement valve apparatus 10 is once again disposed within lumen 112 of sheath 110 of delivery system 100. As seen in Figure 3B, the apparatus is deployed from the lumen and expands to a partially deployed configuration across the patient's native aortic valve **AV.** A separate, expandable embolic filter 200 is also deployed from lumen 112 downstream of apparatus 10 within the patient's aorta **A**, such that the filter sealingly engages the aorta. Any emboli generated during further expansion of apparatus 10 to a fully deployed configuration would be filtered out of the patient's blood stream via the filter and/or lumen 112 of sheath 110. Filter 200 preferably is porous to allow for uninterrupted blood flow through aorta **A** during use of the filter. The filter may, for example, be fabricated from a porous polymer membrane, or from a braid or mesh, e.g. a braided Nitinol structure.

As seen in Figure 3C, balloon catheter 130 may be advanced through sheath 110 and filter 200 into apparatus 10. The balloon may be inflated to further expand apparatus 10 to the fully deployed configuration. Emboli **E** generated during deployment of apparatus 10 then may be captured or otherwise filtered by filter 200. As seen in Figure 3D, balloon catheter 130 then may be deflated and removed from the patient, filter 200 may be collapsed within lumen 112 of sheath 110, and delivery system 100 may be removed, thereby completing the protected valve replacement procedure.

It should be understood that balloon catheter 130 alternatively may be used to perform valvuloplasty prior to placement of apparatus 10 across the diseased valve. In this configuration, filter 200 may be utilized to capture emboli generated during the valvuloplasty procedure and prior to placement of apparatus 10, as well as to provide embolic protection during placement and deployment of the replacement valve apparatus. After the valvuloplasty procedure, apparatus 10 may be deployed with or without balloon catheter 130.

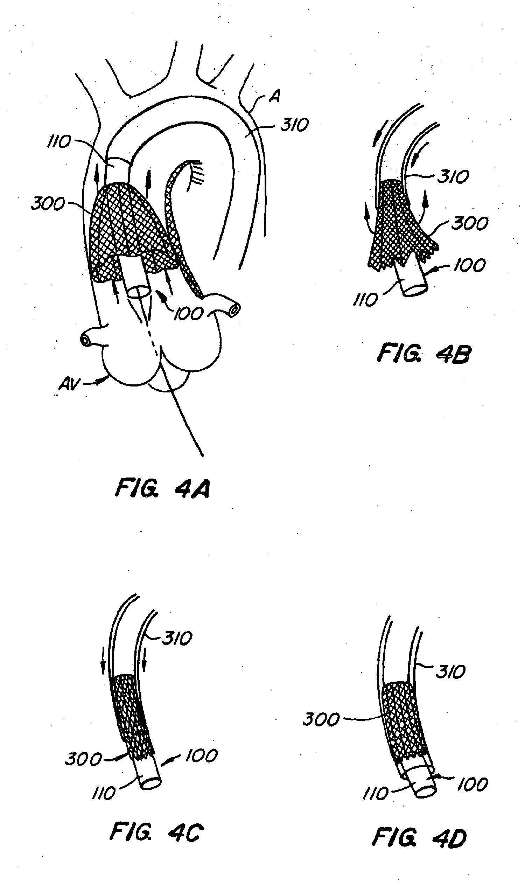

Referring now to Figures 4, yet another method and apparatus for protecting against embolization is described, wherein an embolic filter is coaxially advanced over, or is coupled to, an exterior of a replacement valve delivery catheter. In Figure 4A, replacement valve apparatus, e.g., apparatus 10, is disposed for delivery within the lumen of a delivery sheath, e.g., delivery sheath 110 of delivery system 100. Expandable embolic filter 300 is either coupled to, or is advanceable over, an exterior surface of the delivery sheath.

When filter 300 is advanceable over the delivery sheath, sheath 110 may be positioned in a vicinity of a patient's diseased heart valve, as shown, and filter 300 may be advanced along the exterior of delivery sheath via coaxially-disposed pusher sheath 310. Delivery sheath 110 preferably comprises a motion limitation element, such as a cross-section of locally increased diameter (not shown), which limits advancement of filter 300 relative to the delivery sheath.

When filter 300 is coupled to the exterior of delivery sheath 110, the filter may be collapsed for delivery by advancing pusher sheath 310 over the filter, such that the filter is sandwiched in an annular space between delivery sheath 110 and pusher sheath 310. Replacement valve apparatus 10, delivery system 100, filter 300 and pusher sheath 310 then may be endovascularly advanced to the vicinity of the patient's diseased heart valve **AV.** Once properly positioned, the pusher sheath may be retracted, such that filter 300 dynamically expands into sealing contact with the patient's aorta **A,** as in Figure 4A.

Regardless of whether filter 300 is coupled to, or is advanceable over, delivery sheath 110; once properly positioned, the filter sealingly contacts the patient's aorta and filters blood passing through the aorta to remove any harmful emboli (arrows illustrate blood flow in Figure 4A). Thus, the replacement valve apparatus may be deployed while the filter protects against embolization. As seen in Figure 4B, once embolic protection is no longer desired, e.g., after endovascular replacement of the patient's diseased heart valve, filter 300 may be collapsed for removal by advancing pusher sheath 310 relative to delivery sheath 110 and filter 300. Figure 4B illustrates the filter after partial collapse, while Figure 4C shows the filter nearly completely collapsed. In Figure 4D, filter 300 is fully enclosed within the annular space between delivery sheath 110 and pusher sheath 310. Any dangerous emboli generated during deployment of the replacement valve apparatus are trapped between filter 300 and the exterior surface of delivery sheath 110. Delivery system 100, filter 300 and pusher sheath 310 then may be removed from the patient to complete the procedure.

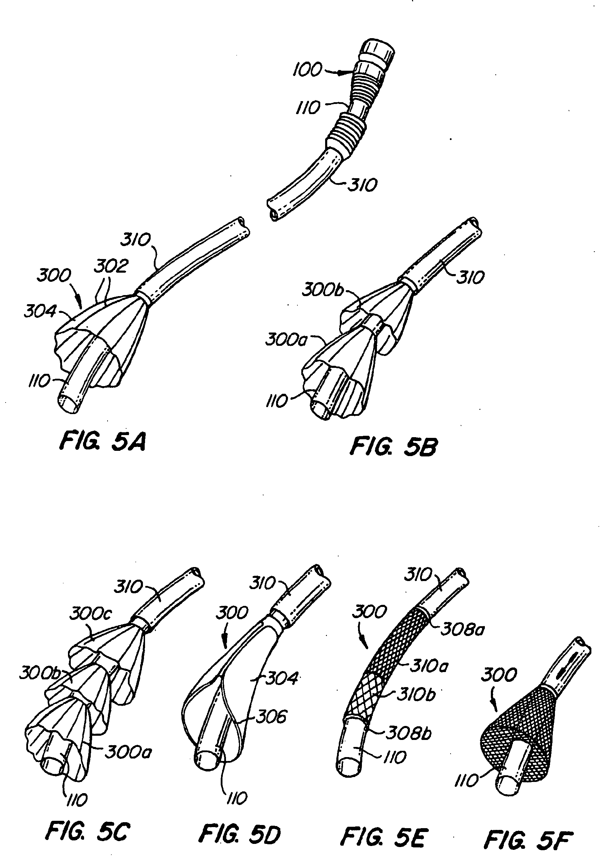

With reference to Figures 5, alternative embodiments of the embolic protection apparatus of Figures 4 are described. In Figure 5A, filter 300 is substantially the same as in Figures 4, but a proximal control region of the embolic protection apparatus, which is disposed outside of the patient, is also described. Region 400, which generally is shown as useable with any of the embodiments of Figure 5, comprises proximal handle 115 of delivery sheath 110, as well as proximal handle 315 of pusher sheath 310. A medical practitioner may grasp handle 115 with a first hand and handle 315 with a second hand for relative movement of pusher sheath 310 and delivery sheath 110.

In Figure 5B, filter 300 comprises first filter 300a and second filter 300b. As with the unitary filter of Figures 4 and 5A, filters 300a and 300b may be coupled to, or advanceable over, the exterior of sheath 110. As another alternative, filter 300a may be coupled to the delivery sheath, while filter 300b is advanceable over the sheath. Filters 300a and 300b may be deployed and retrieved as described previously with respect to Figures 4. Specifically, one or both of the filters may be advanced along delivery sheath 110 via pusher sheath 310, or may be expanded from the annular space between the delivery and pusher sheaths. Likewise, the filters may be collapsed for retrieval within the annular space.

Providing multiple filters may reduce a risk of embolization via emboli inadvertently bypassing the first filter, for example, due to an imperfect seal between the filter and the patient's anatomy. Additionally, each of the filters may have a different porosity; for example, filter 300a may provide a rough filter to remove larger emboli, while filter 300b may comprise a finer porosity to capture smaller emboli. Filtering the emboli through multiple filters may spread the emboli over multiple filters, thereby reducing a risk of impeding blood flow due to clogging of a single filter with too many emboli. The embodiment of Figure 5C extends these concepts: filter 300 comprises first filter 300a, second filter 300b and third filter 300c. As will be apparent, any number of filters may be provided.

The filters of Figures 5A-5C generally comprise expandable baskets having self-expanding ribs 302, e.g., Nitinol or spring steel ribs, surrounded by a porous and/or permeable filter membrane 304. Figure 5D provides an alternative filter 300 comprising a self-expanding wire loop 306 surrounded by membrane 304. Deployment and retrieval of filter 300 of Figure 5D is similar to that of filters 300 of Figures 5A-5C.

Figures 5E and 5F illustrate yet another embodiment of filter 300. In Figure 5E, filter 300 is shown in a collapsed delivery configuration against the exterior surface of delivery sheath 110. Filter 300 is proximally coupled to pusher sheath 310 at attachment point 308a, and is distally coupled to, or motion limited by, delivery sheath 110 at attachment point 308b. Filter 300 comprises proximal braid 310a and distal braid 310b, e.g., proximal and distal Nitinol braids. The proximal braid preferably comprises a tighter weave for filtering smaller emboli, and may also be covered by a permeable/porous membrane (not shown). Distal braid 310b comprises a more open braid to facilitate expansion, as well as capture of larger emboli.

In Figure 5F, pusher sheath 310 has been advanced relative to delivery sheath 110, thereby expanding filter 300 for capturing emboli. Once embolic protection is no longer desired, e.g., after endovascular replacement of the patient's diseased heart valve, pusher sheath 310 may be retracted relative to the delivery sheath, which collapses the filter back to the delivery configuration of Figure 5E and captures emboli between the filter and the delivery sheath. As another alternative, pusher sheath 310 may be further advanced relative to the delivery sheath, thereby collapsing the filter into a retrieval configuration wherein the proximal braid covers the distal braid (not shown).

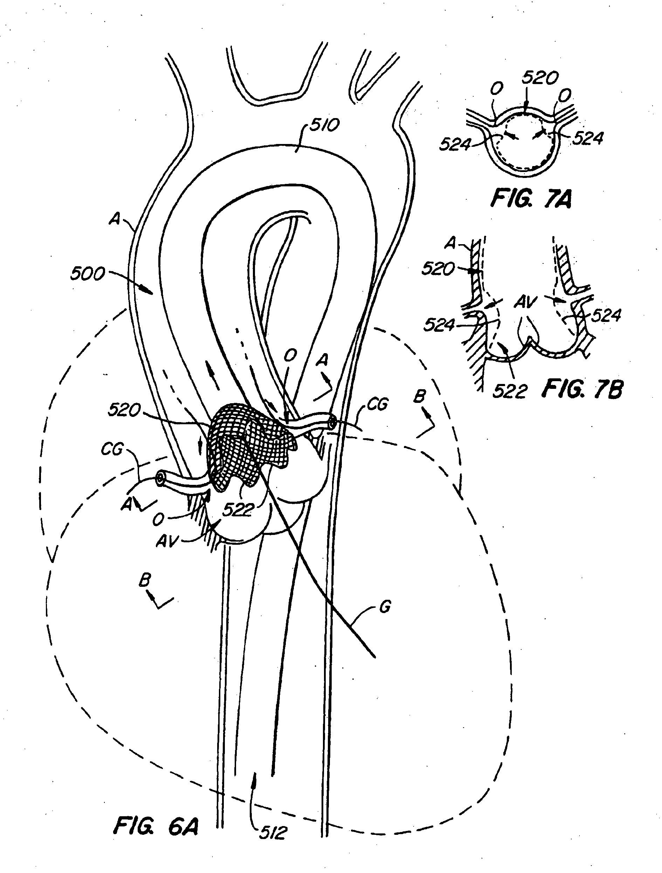

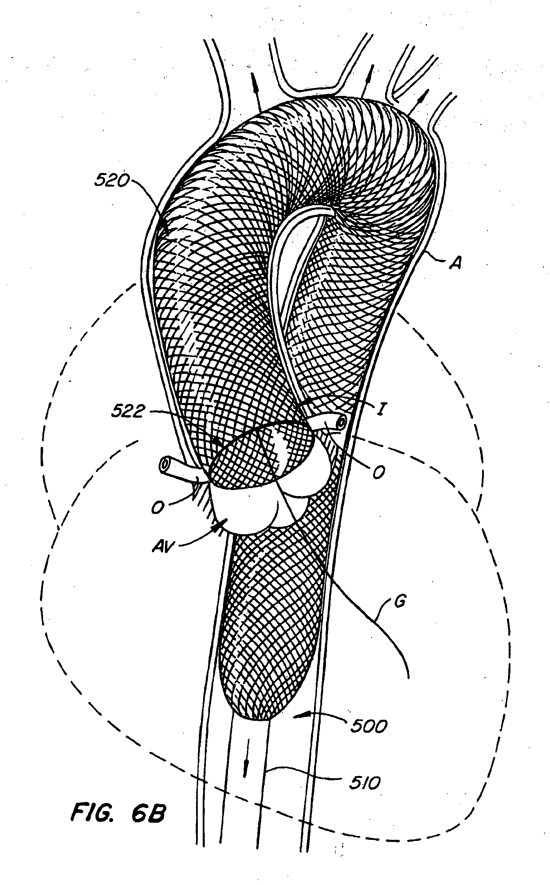

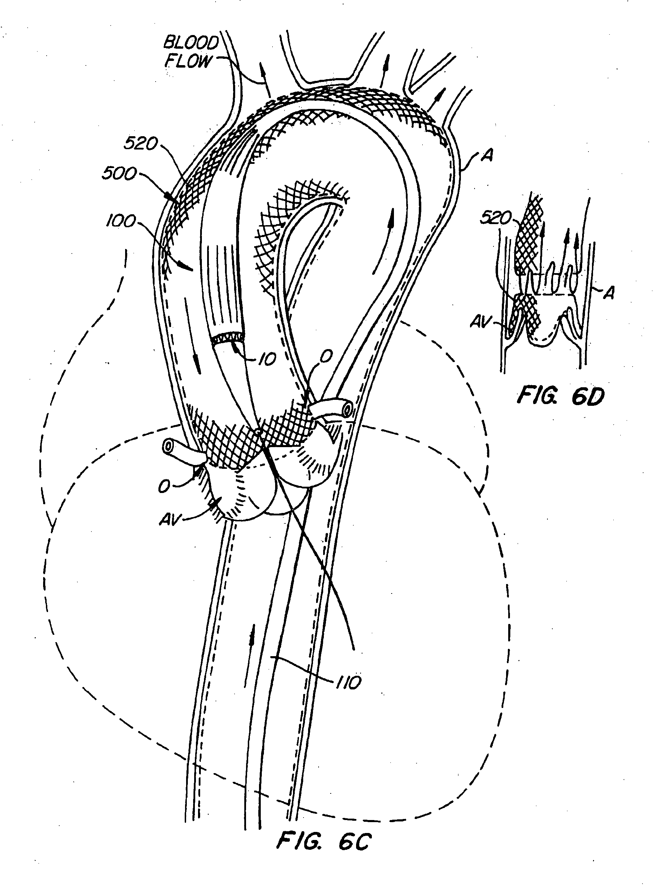

Referring now to Figures 6, another method and apparatus for protecting against embolization is described. In Figure 6A, guidewire G has been percutaneously advanced through a patient's aorta **A,** past the patient's diseased aortic valve **AV** and into the left ventricle. Coronary guidewires **CG** may also be provided to facilitate proper positioning of elements advanced over guidewire **G.**

Embolic protection system 500 has been endovascularly advanced over guidewire **G** to the vicinity of the patient's aortic valve **AV.** System 500 includes exterior sheath 510 and embolic filter 520. The embolic filter may be collapsed for delivery and/or retrieval within lumen 512 of the sheath. As seen in Figures 6A and 6B, exterior sheath 510 may be withdrawn relative to filter 520, such that the filter self-expands into contact with the patient's anatomy. The open mesh of the braid, e.g. Nitinol braid, from which the filter is fabricated, provides filtered perfusion: filtered blood continues to flow through the filter and through the patient's aorta, as well as through side-branchings off of the aorta. Optionally, filter 520 may also comprise a permeable/porous membrane to assist filtering.

As shown in Figure 6A, filter 520 optionally may comprise a scalloped distal edge 522 that fits behind the valve leaflets and over the leaflet commissures of aortic valve **AV**. The depth, number and/or shape(s) of distal edge 522 may be specified, as desired. Furthermore, marking indicia **I** (see Figure 6B) may be provided on or near the edge to facilitate proper alignment of the edge with the patient's coronary ostia **O*.** Figure 6B illustrates an alternative embodiment of the filter wherein distal edge 522 is substantially planar. This may simplify placement of the filter without requiring complicated alignment with the patient's coronary ostia **O,** and the planar distal edge may simply rest on or near the valve leaflet commissures.

In addition to providing embolic protection, filter 520 may aid delivery of replacement valve apparatus. As seen in Figure 6B, filter 520 contacts the inner wall of aorta A over a significant distance, thereby providing a non-slip protective layer for guiding additional catheters past blood vessel branches without damaging the vessel walls. As seen in the cutaway view of Figure 6C, delivery system 100, having replacement valve apparatus 10 disposed therein, may then be advanced through embolic protection system 500; and endovascular, beating-heart replacement of the patient's diseased aortic valve **AV** may proceed in an embolically protected manner. As will be apparent, any alternative replacement valve apparatus and delivery system may be used in combination with embolic protection system 500. Furthermore, as seen in the detail view of Figure 6D, all or part of filter 520 may be detachable and remain as part of the implanted replacement valve apparatus, e.g., as an anchor for the replacement valve.

Referring now to Figures 7, optional end geometry for filter 520 is described. As seen in Figure 7B, distal edge 522 of filter 520 may distally extend into the cusps of the patient's diseased valve, for example, as a means to reference distances and/or to ensure full engagement. In order to guarantee adequate blood flow to the patient's coronary arteries, filter 520 may comprise heat-set or otherwise-formed indentations 524 that increase surface area flow through the filter to the patient's coronary arteries. The indentations may also aid proper alignment of the replacement valve apparatus, e.g., may be used in conjunction with coronary guidewires **CG**.

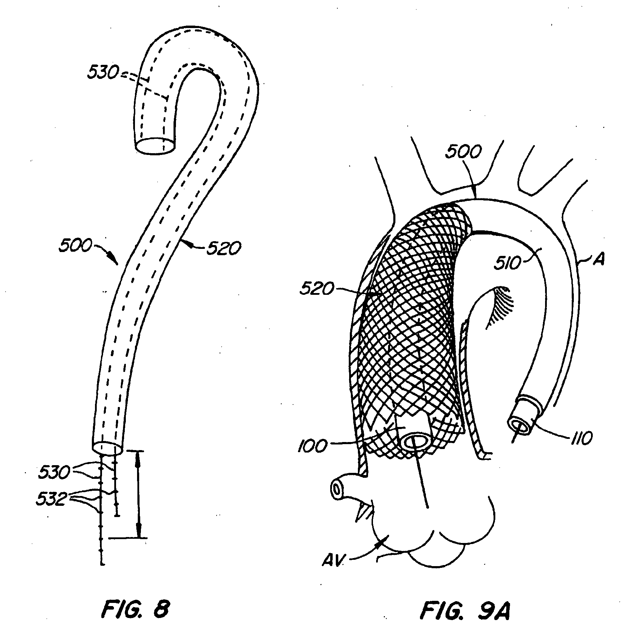

With reference to Figure 8, an embodiment of embolic protection apparatus 500 is described comprising a measuring element. Embolic filter 520 may, for example, comprise a pair of opposed thin wires 530 that are anchored to the distal end of the filter and extend out the other end to provide a measuring element. The wires optionally may be radiopaque to facilitate visualization. Wires 530 comprise measurement indicia 532 on their proximal ends that give the distance between the indicia and the distal end of the wire. The average distance measured between the two wires provides the center axis distance through the patient's aorta to the valve commissures.

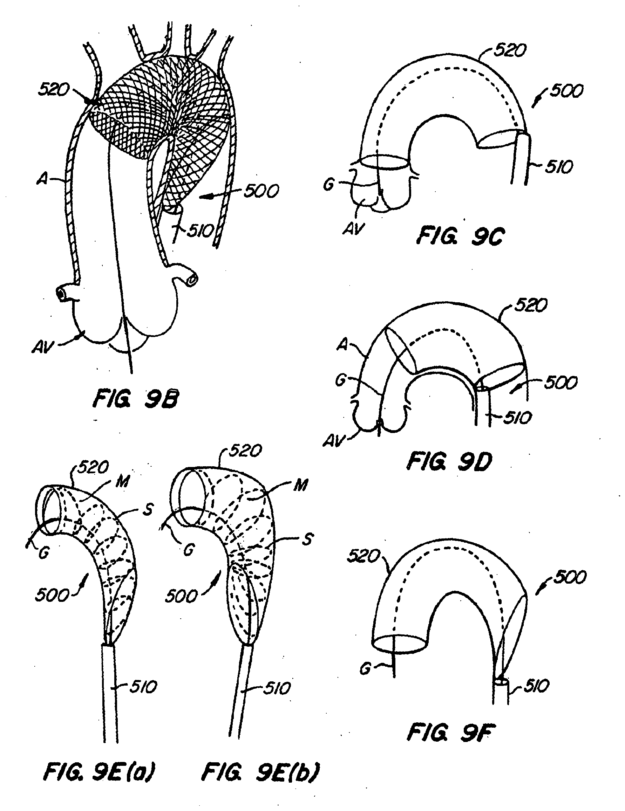

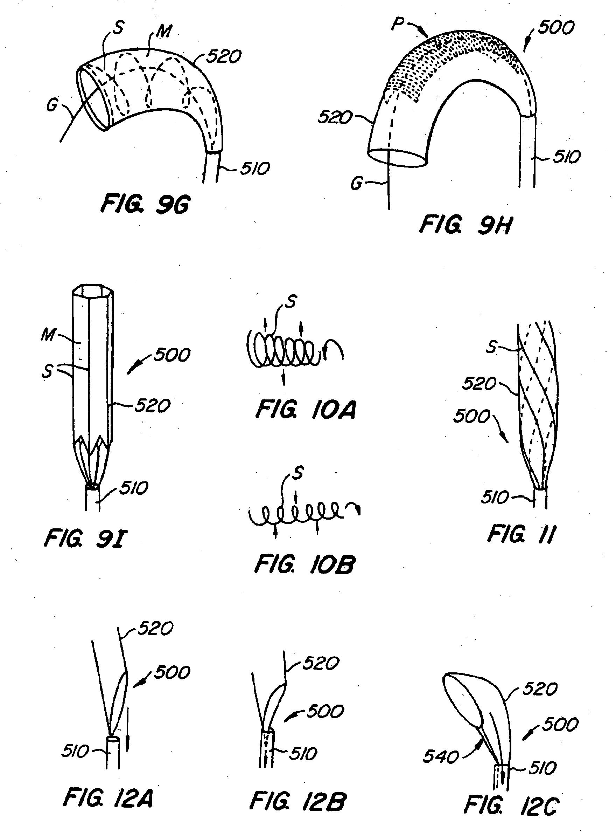

Referring now to Figures 9, various exemplary alternative embodiments of embolic protection system 500 are described. In Figure 9A, a shorter version of embolic filter 520 is shown. The filter is disposed in the annular space between exterior sheath 510 and delivery system 100/replacement valve apparatus 10. The filter may be fabricated in a shorter length, or may be only partially deployed to a desired length.

Figure 9B illustrates another optionally short-necked version of filter 520. However, unlike the filter of Figure 9A, the proximal end of filter 520 in Figure 9B is at least partially disconnected from sheath 510. Thus, filter 520 is a diverter that diverts emboli past the primary upper circulatory branchings of aorta **A**, e.g., those leading to the patient's carotid arteries, thereby protecting the patient from cerebral embolization. The emboli then may be allowed to continue downstream to less critical and/or dangerous regions of the patient's anatomy.

Optionally, suction may be applied through the lumen of sheath 510 to remove at least a portion of the emboli from the patient. Alternatively, a stand-alone suction catheter (not shown) may be advanced over, through or alongside sheath 510 to the vicinity of, or within, filter 520; suction then may be drawn through the suction catheter to aspirate the emboli. The suction catheter optionally may be part of delivery system 100, e.g., sheath 110.

The proximal end of filter 520 illustratively comprises a tapered or angled opening to facilitate collapse and removal of the filter from the patient. The distal end of the filter may likewise be tapered or angled in any desired direction or configuration.

In Figure 9B, replacement valve apparatus optionally may be deployed directly through sheath 510 without an intervening delivery sheath. Alternatively, a delivery sheath, such as sheath 110, may be provided, as described previously. The delivery sheath may be advanced through or adjacent to filter sheath 510; alternatively, sheath 510 may be removed during placement of the replacement valve apparatus.

Figure 9C illustrates an alternative embodiment of filter 520 wherein the filter comprises a permeable or porous membrane, web, film, etc., as opposed to a braid. The membrane may comprise a specified porosity, for example, pores of about 100 µm or less. In Figure 9C, the proximal opening of filter 520 has been squared off. Figure 9D illustrates an embodiment wherein sheath 510 is disposed along the opposing side of the patient's aorta **A**, as compared to the embodiment of Figure 9C.

In Figure 9E, filter 520 comprises membrane **M** with reinforcing, spiral-wound support S. The support optionally may be disposed within a guide track of the membrane and may be advanced or retracted within the membrane, as desired. Figure 9E illustratively shows the proximal end of filter 520 tapered or angled in two different configurations; in Figure 9E(a), the taper distally extends towards the lesser curvature of the aorta, while in Figure 9E(b), the taper distally extends towards the greater curvature. Additional configurations will be apparent.

Figure 9F illustrates a membrane embodiment of filter 520, which is similar to the braid embodiment of Figure 9B. Figure 9G illustrates another membrane/spiral-wound embodiment of filter 520. However, the filter of Figure 9G is proximally attached to sheath 510, such that embolic particles are captured and removed from the patient, rather than diverted. Figure 9H provides another proximally attached embodiment of the filter having one or more regions of specially designed porosity **P**. For example, the size and/or density of the pores may be varied as desired in the vicinity of vessel branchings, e.g., to enhance blood flow and/or to more finely filter particles.

Filter 520 may have a biased profile, e.g., such that it naturally assumes the curve of the patient's aorta. Alternatively, the filter may comprise a non-biased or straight profile as in Figure 9I, which may be urged into a curved configuration. In Figure 9I, filter 520 comprises membrane **M** strung between longitudinal support structure **S**.

Referring now to Figures 10, a spiral wound structure for use with any of the previously described filters is described. Structure S acts as a radially-expansive support when torqued in a first direction, as seen in Figure 10A. When torqued in the opposing direction, the structure loosens and contracts in diameter, as seen in Figure 10B. The torque characteristics of structure S may be utilized to expand and contract an embolic filter, as well as to capture emboli disposed within the filter.

As shown in Figure 11, filter 520 may comprise multiple longitudinal supports wound in long spirals. The supports may increase hoop strength. They may also help maintain a desired length of the filter.

Figures 12 illustrate alternative deployment and retrieval methods for filter 520. In Figure 12A, the proximal end of filter 520 is attached to the distal end of sheath 510. The filter and sheath may be advanced and withdrawn together with the filter conforming to the patient's anatomy as it is it repositioned. Alternatively, an additional over-sheath may be provided for collapsing the filter to a reduced delivery and retrieval configuration.

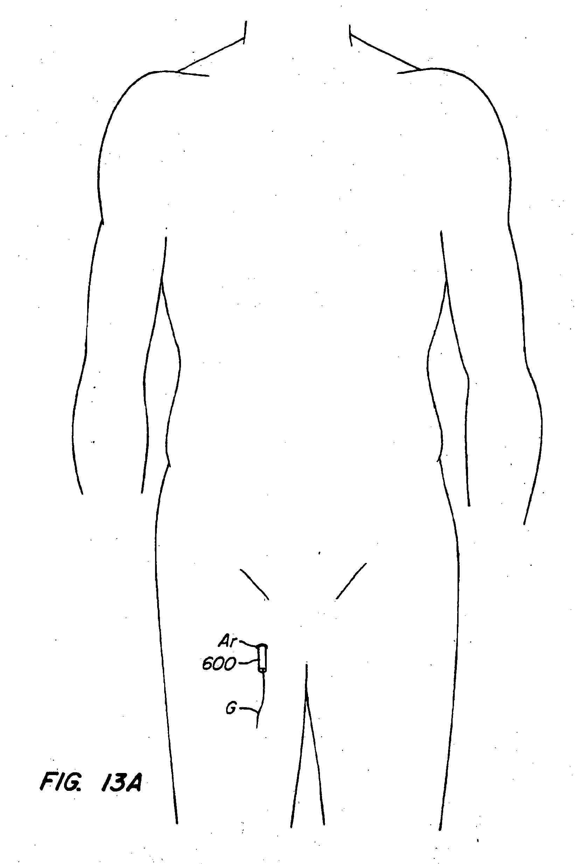

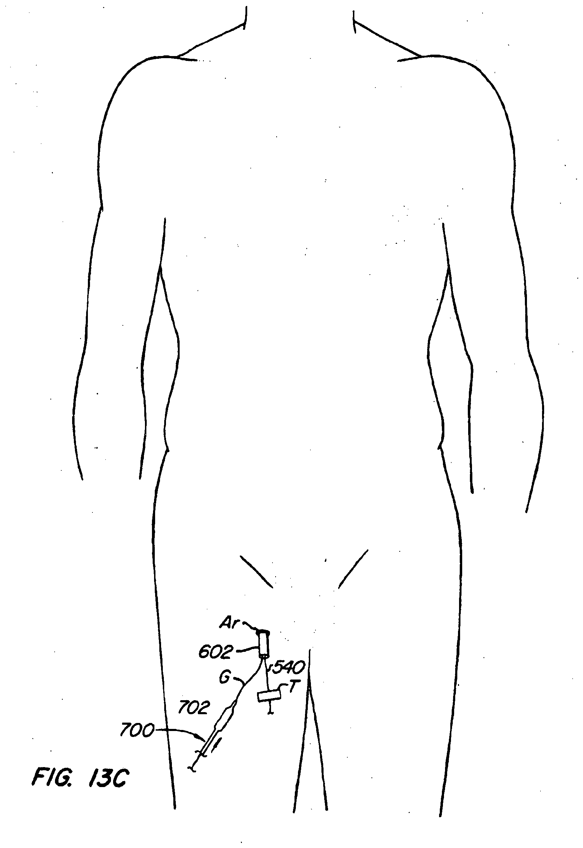

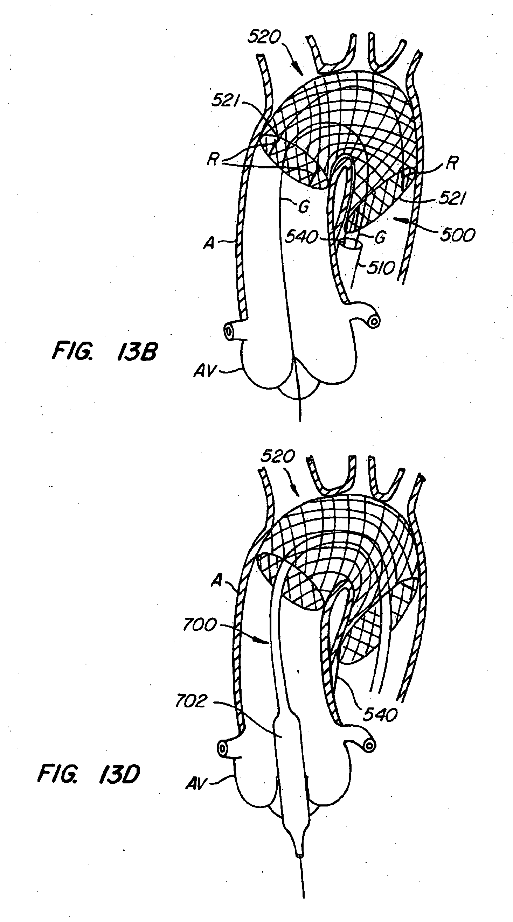

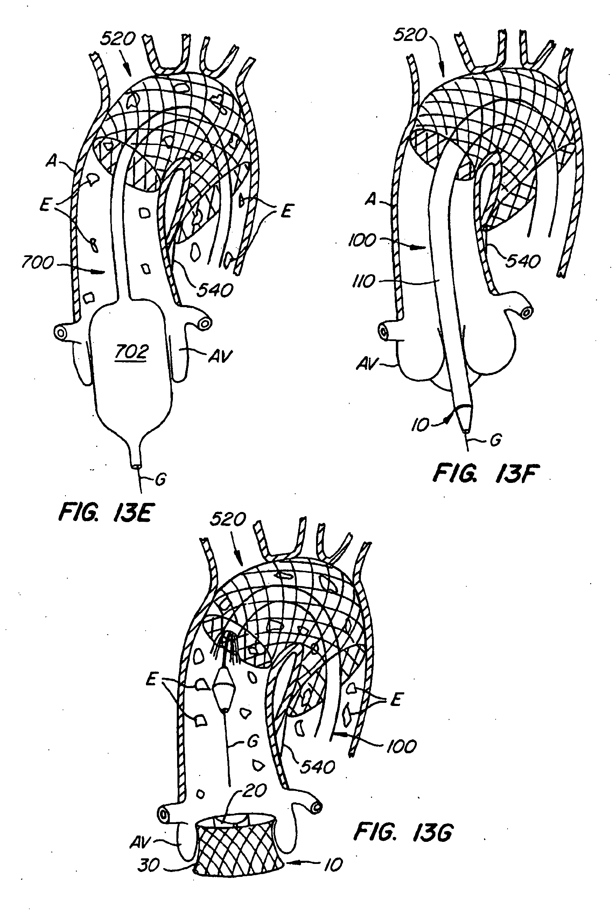

As seen in Figure 12B, filter 520 alternatively may be collapsed within sheath 510 during delivery and retrieval, e.g. via a pullwire coupled to a proximal end of the filter (see Figures 13). As seen in Figure 12C, embolic protection system 500 optionally may comprise pullwire 540 attached to the distal outlet of filter 520. By keeping the wire taut during retrieval of filter 520, it is expected that a risk of snagging, or otherwise hanging up, filter 520 on sheath 510 will be reduced.

Prior to implantation of a replacement valve, such as those described above, it may be desirable to perform a valvuloplasty on the diseased valve by inserting a balloon into the valve and expanding it, e.g., using saline mixed with a contrast agent. In addition to preparing the valve site for implantation, fluoroscopic viewing of the valvuloplasty will help determine the appropriate size of replacement valve implant to use. During valvuloplasty, embolic protection, e.g., utilizing any of the embolic filters described previously, may be provided.

Referring now to Figures 13, a method of replacing a patient's diseased aortic valve utilizing replacement valve apparatus 10 and delivery system 100, in combination with a diverter embodiment of embolic protection system 500, is described. Although a retrograde approach via the femoral artery illustratively is utilized, it should be understood that alternative approaches may be utilized, including, but not limited to, radial or carotid approaches, as well as trans-septal antegrade venous approaches.

As seen in Figure 13A, arteriotomy puncture site Ar is formed, and introducer sheath 600 is advanced in a minimally invasive fashion into the patient's femoral artery. The introducer preferably initially comprises a relatively small sheath, for example, an introducer sheath on the order of about 6 Fr-compatible. Guidewire **G** is advanced through the introducer sheath into the femoral artery, and is then further advanced through the patient's aorta and across the patient's diseased aortic valve.

Additionally, imaging may be performed to determine whether the patient is a candidate for valvuloplasty and/or endovascular valve replacement. For example, angiographic imaging, *per se* known, may be performed via an angiography catheter (not shown) advanced from a femoral, radial, or other appropriate entry site. The angiography catheter may, for example, have a profile on the order of about 5 Fr to 8 Fr, although any alternative size may be used.

If it is determined that the patient is not a candidate for valvuloplasty and/or endovascular valve replacement, the guidewire and introducer sheath (as well as any imaging apparatus, e.g., the angiography catheter) may be removed from the patient, and the arteriotomy site may be sealed. If it is determined that the patient is a candidate, the arteriotomy site may be expanded, and, upon removal of any imaging apparatus, introducer sheath 600 may be exchanged with a larger introducer sheath 602 (see Figure 13C), for example, an introducer sheath on the order of about 14 Fr compatible, to facilitate endovascular valvuloplasty and/or valve replacement.

As seen in Figure 13B, embolic protection system 500 then may be advanced over guidewire **G** to the vicinity of the patient's diseased valve. Sheath 510 may be retracted relative to diverter filter 520, such that the diverter filter, which preferably comprises a self-expanding wire braid, expands into contact with the wall of aorta **A** downstream of aortic valve **AV.** Sheath 510 of embolic protection system 500 then may be removed from the patient.

Filter 520 is configured to divert emboli, generated during endovascular treatment of valve **AV**, away from the patient's cerebral vasculature. The filter illustratively comprises optional proximal and distal interfaces 521 of enlarged diameter that contact the wall of aorta **A,** while a central section of the filter disposed between the interfaces moves freely or 'floats' without engaging the aorta. This may reduce friction during deployment and/or retrieval of the filter, and may also reduce damage caused by the filter to the wall of the aorta. Filter 520 alternatively may contact aorta **A** along its length, as in Figures 13D-13G. Filter 520 also optionally may comprise internal rails **R** that may be used to guide endovascular treatment tools through the filter. Filter 520 illustratively is coupled proximally to pullwire 540, which extends from the proximal end of the filter to the exterior of the patient. Pullwire 540 allows a medical practitioner to maneuver filter 520, as desired.

As seen in Figure 13C, upon removal of sheath 510 from the patient, guidewire **G** and pullwire 540 extend through introducer sheath 602. Advantageously, with filter 520 positioned as desired within the patient's aorta and with slack removed from pullwire 540, the filter may be maintained at the desired position by reversibly maintaining the position of pullwire 540, e.g., by reversibly attaching the pullwire to the exterior of the patient via surgical tape **T.** In this manner, a medical practitioner may properly position diverter filter 520, then leave it in the desired position without requiring significant manipulation or monitoring during endovascular treatment of the patient's diseased aortic valve **AV**. The open proximal end of diverter filter 520 allows additional endovascular tools, such as valvuloplasty catheter 700 and/or replacement valve apparatus 10 disposed within delivery system 100, to be advanced through the diverter.

In Figures 13C and 13D, optional valvuloplasty catheter 700, having expandable balloon 702, is advanced over guidewire **G** and through introducer sheath 602 into the patient's vasculature. Catheter 700 preferably comprises a delivery profile on the order of about 8-16 Fr, while balloon 702 preferably comprises an expanded diameter on the order of about 18 mm to 30 mm, more preferably about 20 mm to 23 mm. Proper sizing of balloon 702 optionally may be determined, for example, via angiographic imaging of aortic valve **AV.**

Balloon 702 is endovascularly advanced through aorta **A** and diverter filter 520 across diseased aortic valve **AV.** Diverter filter 520 advantageously guides catheter 700 past the arterial branches of aorta **A** as the catheter passes through the filter. In this manner, filter 520 facilitates proper placement of balloon 702, while reducing a risk of injury to the arterial branches.

In Figure 13E, once positioned across the aortic valve, balloon 702 is expanded to break up or otherwise crack calcification and/or lesion(s) along the valve. Expansion may, for example, be achieved using saline mixed with a contrast agent. In addition to preparing the valve site for implantation, fluoroscopic viewing of the contrast agent and the valvuloplasty may help determine the appropriate size of replacement valve apparatus 10 to use. Balloon 702 is then deflated, and valvuloplasty catheter 700 is removed from the patient. Emboli **E** generated during valvuloplasty travel downstream through aorta **A,** where they are diverted by filter 520 away from the patient's cerebral vasculature.

Optionally, multiple catheters 700 may be provided and used sequentially to perform valvuloplasty. Alternatively or additionally, multiple catheters 700 may be used in parallel (e.g., via a 'kissing balloon' technique). The multiple catheters may comprise balloons 702 of the same size or of different sizes.

After optionally performing valvuloplasty, aortic valve **AV** may once again be imaged, e.g. via fluoroscopy and angiography, to determine whether the patient is a candidate for endovascular valve replacement. If it is determined that the patient is not a candidate, embolic protection system 500, as well as guidewire **G** and introducer sheath 602, may be removed from the patient, and arteriotomy site **AR** may be sealed. A suction catheter optionally may be positioned within filter 520 prior to retrieval of the filter to 'vacuum out' any emboli caught therein.

In order to collapse filter 520 for retrieval, sheath 510 of embolic protection system 500 optionally may be re-advanced through introducer 602 and over pullwire 540 (optionally, also over guidewire **G**) to contact a proximal region of the filter (see Figures 12). The tapered proximal region may function as collapse element that facilitates sheathing of filter 520 for delivery and/or retrieval, e.g., by distributing forces applied to the filter by sheath 510 along a greater longitudinal length of the filter, as compared, for example, to embodiments of the filter that are not proximally tapered. Additional and alternative collapse elements may be provided with filter 520 or with sheath 510. The collapse element may collapse the filter, e.g., by collapsing the filter braid.

Filter 520 alternatively may be retrieved by proximally retracting pullwire 540 without collapsing the filter within a retrieval sheath, thereby proximally retracting filter 520 directly through the patient's vasculature. As yet another alternative, a specialized retrieval sheath, e.g., a sheath of larger or smaller profile than sheath 510, may be utilized. The retrieval sheath optionally may comprise a distally enlarged lumen to accommodate the collapsed filter.

In Figure 13F, if it is determined that the patient is a candidate for endovascular valve replacement, delivery system 100, having replacement valve apparatus 10 disposed therein in a collapsed delivery configuration, may be endovascularly advanced over guidewire **G** through the introducer sheath, through filter 520 and across the patient's aortic valve **AV.** As during advancement of balloon catheter 700, diverter filter 520 advantageously guides delivery system 100 past arterial branches of aorta **A**, while the delivery system is advanced through the filter. In this manner, filter 520 facilitates proper positioning of apparatus 10, while protecting the aortic side branches from injury.

As it is expected that delivery system 100 may have a delivery profile on the order of about 18-21 Fr, preferably about 19 Fr, introducer sheath 602 optionally may be exchanged for a larger introducer sheath in order to accommodate the delivery system. Alternatively, in order to reduce the size of arteriotomy site **AR,** it may be desirable to remove the introducer sheath and to advance delivery system 100 directly through the arteriotomy site without an intervening introducer sheath, such that sheath 110 of the delivery system acts as the introducer sheath. Delivery system 100 optionally may comprise a rapid-exchange lumen for advancement over guidewire **G**.

If introducer sheath 602 is exchanged or removed, pullwire 540 temporarily may be disconnected from the exterior of the patient, e.g., by removing tape **T.** The introducer sheath then optionally may be removed or exchanged, and pullwire 540 may be re-affixed to the patient. During removal and/or exchange of introducer sheath 602 (i.e., while pullwire 540 is not affixed to the patient), a medical practitioner preferably grasps pullwire 540 and maintains its position relative to arteriotomy site **AR,** thereby maintaining the position of filter 520 deployed within the patient.

In Figure 13G, once replacement valve apparatus 10 has been properly positioned across the patient's diseased aortic valve **AV,** sheath 110 of delivery system 100 may be retracted, and apparatus 10 may be deployed as described previously, thereby endovascularly replacing the patient's diseased valve. Emboli **E** generated during deployment of apparatus 10 are diverted away from the patient's carotid arteries and cerebral vasculature by filter 520. Delivery system 100 then may be removed from the patient.

Filter 520 optionally may be vacuumed out via a suction catheter, e.g., suction drawn through sheath 110. Filter 520 and guidewire **G** then may be removed from the patient as discussed previously, and arteriotomy site **AR** may be sealed to complete the procedure. Guidewire **G** may retrieved and removed before, during or after retrieval and removal of filter 520. Retrieval and removal of the filter may comprise reintroduction of sheath 510 (e.g., over pullwire 540 and directly through the arteriotomy site, through an introducer sheath or through sheath 110 of delivery system 100) and collapse of filter 520 within the sheath. Alternatively, removal of filter 520 may comprise retraction of pullwire 540 without collapse of the filter in an intervening retrieval sheath. Sealing of the arteriotomy site may comprise any known sealing method, including, but not limited to, application of pressure, introduction of sealants, suturing, clipping and/or placement of a collagen plug.

In Figures 13, although diversion and/or filtering of emboli illustratively has been conducted during both valvuloplasty and endovascular deployment of replacement valve apparatus, it should be understood that such diversion/filtering alternatively may be performed only during valvuloplasty or only during endovascular valve replacement. Furthermore, it should be understood that embolic protection may be provided during deployment of any endovascular replacement valve apparatus and is not limited to deployment of the specific embodiments of such apparatus described herein.

## Claims

1. Apparatus for protecting against embolization during endovascular replacement of a patient's heart valve, the apparatus comprising:
a replacement valve (20) configured for endovascular delivery and deployment; and
an embolic protection system (500) including an exterior sheath (510) and an embolic filter (520), wherein the embolic filter (520) is configured for disposal downstream of the replacement valve (20) during deployment of the valve,
**characterized in that**
the embolic filter (520) is configured for expansion from a collapsed delivery configuration to an expanded deployed configuration, wherein the embolic filter (520) is configured to be collapsed for delivery and/or retrieval within lumen (512) of the sheath (510), wherein the proximal end of the filter (520) is at least partially disconnected from the sheath (510), thereby diverting emboli away from the patient's cerebral vasculature without capturing the emboli within the filter (520).

2. The apparatus of claim 1, wherein the embolic filter (520) is coupled to the replacement valve (20).

3. The apparatus of claim 1, wherein the embolic filter (520) is decoupled from the replacement valve (20).

4. The apparatus of claim 1, wherein the embolic filter (520) is configured to contact a patient's aorta and form a circumferential seal against the aorta in the deployed configuration.

5. The apparatus of claim 1, wherein the embolic filter (520) is configured for endovascular delivery in the collapsed delivery configuration.

6. The apparatus of claim 1, wherein the replacement valve (20) is configured for endovascular delivery through the embolic filter (520).

7. The apparatus of claim 1 further comprising a suction element configured to aspirate diverted emboli from the patient's bloodstream.

8. The apparatus of claim 1, wherein the embolic filter (520) is fabricated from an expandable wire braid or mesh.

9. The apparatus of claim 1, wherein the embolic filter (520) comprises a spiral-wound structure.

10. The apparatus of claim 9, wherein the spiral-wound structure is configured to expand when torqued in a first direction and to contract when torqued in an opposite direction.

11. The apparatus of claim 1, wherein the embolic filter (520) comprises a permeable membrane having a specified porosity.

12. The apparatus of claim 11, wherein the specified porosity comprises pores less than about 100 µm in diameter.

13. The apparatus of claim 11, wherein the permeable membrane comprises a varying porosity.

14. The apparatus of claim 1 further comprising an expandable balloon (702) for performing valvuloplasty, wherein the embolic filter (520) is configured to divert emboli generated during valvuloplasty.

15. The apparatus of claim 1, wherein the embolic filter (520) comprises at least one measuring element for determining distances within the patient.

16. The apparatus of claim 16, wherein the measuring element is configured to provide a center-axis distance between the patient's heart valve and a desired location within the patient's aorta.

17. The apparatus of claim 1, wherein the embolic filter (520) comprises a curved profile in the deployed configuration.

18. The apparatus of claim 1, wherein the embolic filter (520) is configured for collapse from the expanded deployed configuration to a collapsed retrieval configuration.

19. The apparatus of claim 18 further comprising a collapse element adapted to facilitate collapse and retrieval of the filter (520) from the patient.

20. The apparatus of claim 19, wherein the embolic filter (520) comprises the collapse element.

21. The apparatus of claim 20, wherein the collapse element comprises a tapered opening disposed at a proximal end of the embolic filter (520).

22. The apparatus of claim 19, wherein the collapse element comprises a retrieval sheath advanceable over the filter (520).

23. The apparatus of claim 4, wherein the embolic filter (520) further comprises proximal and distal interfaces (521), and wherein the embolic filter is configured to contact the patient's aorta only along the proximal and distal interfaces (521).

24. The apparatus of claim 6, wherein the embolic filter (520) is configured to guide a catheter from a proximal end of the filter (520) to a distal end of the filter (520).

25. The apparatus of claim 8, wherein the expandable wire braid or mesh further comprises a Nitinol wire braid or mesh.

26. A kit for endovascularly replacing a patient's diseased heart valve, the kit comprising:
a valvuloplasty balloon catheter (702); and
an apparatus comprising:
an expandable replacement valve (20) configured for endovascular delivery and deployment across the patient's diseased valve; and
an embolic protection system (500) including an exterior sheath (510) and an embolic filter (520), wherein the embolic filter (520) is configured for endovascular delivery and deployment downstream of the patient's diseased valve,
**characterized in that**
the embolic filter (520) is configured for expansion from a collapsed delivery configuration to an expanded deployed configuration, wherein the embolic filter (520) is configured to be collapsed for delivery and/or retrieval within a lumen (512) of the sheath (510), wherein the proximal end of the filter (520) is at least partially disconnected from the sheath (510), thereby diverting emboli generated during valvuloplasty or deployment of the replacement valve.

27. The kit of claim 26 further comprising a delivery system for endovascularly delivering and deploying the expandable replacement valve (20).

28. The kit of claim 26 further comprising a delivery system for endovascularly delivering and deploying the embolic filter (520).

## Patentansprüche

1. Gerät zum Schutz vor Embolisierung während des endovaskulären Ersatzes der Herzklappe eines Patienten, wobei das Gerät umfasst:
eine Ersatzklappe (20) zur endovaskulären Abgabe und zum Einsetzen; und
ein Embolieschutzsystem (500) mit einer Außenhülle (510) und einem Emboliefilter (520), worin der Emboliefilter (520) stromabwärts von der Ersatzklappe (20) während des Einsetzens der Klappe angeordnet werden kann, **dadurch gekennzeichnet, dass**
der Emboliefilter (520) aus einer kollabierten Abgabekonfiguration in eine expandierte eingesetzte Konfiguration expandiert werden kann, worin der Emboliefilter (520) zur Abgabe und/oder zur Rückholung im Lumen (512) der Hülle (510) kollabiert werden kann, worin das proximale Ende des Filters (520) zumindest teilweise von der Hülle (510) gelöst ist, wodurch Emboli von den Zerebralgefäßen des Patienten weg umgelenkt werden können, ohne die Emboli im Filter (520) zu fangen.

2. Gerät nach Anspruch 1, worin der Emboliefilter (520) mit der Ersatzklappe (20) verbunden ist.

3. Gerät nach Anspruch 1, worin der Emboliefilter (520) von der Ersatzklappe (20) gelöst ist.

4. Gerät nach Anspruch 1, worin der Emboliefilter (520) mit der Aorta eines Patienten in Berührung kommen kann und in der eingesetzten Konfiguration eine Umfangsdichtung gegen die Aorta bilden kann.

5. Gerät nach Anspruch 1, worin der Emboliefilter (520) zur endovaskulären Abgabe in der kollabierten Abgabekonfiguration konfiguriert ist.

6. Gerät nach Anspruch 1, worin die Ersatzklappe (20) zur endovaskulären Abgabe durch den Emboliefilter (520) konfiguriert ist.

7. Gerät nach Anspruch 1, das ferner ein Saugelement zur Aspiration von umgelenkten Emboli aus dem Blutstrom eines Patienten umfasst.

8. Gerät nach Anspruch 1, worin der Emboliefilter (520) aus einem expandierbaren Drahtgeflecht oder Netz geformt ist.

9. Gerät nach Anspruch 1, worin der Emboliefilter (520) eine spiralförmig gewickelte Konstruktion umfasst.

10. Gerät nach Anspruch 9, worin die spiralförmig gewickelte Konstruktion expandieren kann, wenn sie in einer ersten Richtung festgezogen wird, und kontrahieren kann, wenn sie in einer entgegengesetzten Richtung festgezogen wird.

11. Gerät nach Anspruch 1, worin der Emboliefilter (520) eine permeable Membran mit einer vorgegebenen Porosität umfasst.

12. Gerät nach Anspruch 11, worin die vorgegebene Porosität Poren mit einem Durchmesser unter 100 µm umfasst.

13. Gerät nach Anspruch 11, worin die permeable Membran eine variierende Porosität umfasst.

14. Gerät nach Anspruch 1, das ferner einen expandierbaren Ballon (702) zur Durchführung einer Valvuloplastik umfasst, worin der Emboliefilter (520) Emboli, die während der Valvuloplastik erzeugt werden, umlenken kann.

15. Gerät nach Anspruch 1, worin der Emboliefilter (520) zumindest ein Messelement zur Ermittlung von Abständen im Patienten umfasst.

16. Gerät nach Anspruch 16, worin das Messelement zur Lieferung eines Mittelachsenabstands zwischen der Herzklappe des Patienten und einer gewünschten Stelle in der Aorta des Patienten konfiguriert ist.

17. Gerät nach Anspruch 1, worin der Emboliefilter (520) in der eingesetzten Konfiguration ein gebogenes Profil umfasst.

18. Gerät nach Anspruch 1, worin der Emboliefilter (520) aus der expandierten eingesetzten Konfiguration in eine kollabierte Rückholkonfiguration kollabieren kann.

19. Gerät nach Anspruch 18, das ferner ein Kollabierelement zur Erleichterung der Kollabierung und Rückholung des Filters (520) aus dem Patienten umfasst.

20. Gerät nach Anspruch 19, worin der Emboliefilter (520) das Kollabierelement umfasst.

21. Gerät nach Anspruch 20, worin das Kollabierelement eine verjüngte Öffnung umfasst, die an einem proximalen Ende des Emboliefilters (520) angeordnet ist.

22. Gerät nach Anspruch 19, worin das Kollabierelement eine Rückholhülle umfasst, die über den Filter (520) vorgeschoben werden kann.

23. Gerät nach Anspruch 4, worin der Emboliefilter (520) ferner proximale und distale Schnittstellen (521) umfasst und worin der Emboliefilter nur entlang der proximalen und distalen Schnittstellen (521) mit der Aorta des Patienten in Berührung kommen kann.

24. Gerät nach Anspruch 6, worin der Emboliefilter (520) einen Katheter von einem proximalen Ende des Filters (520) zu einem distalen Ende des Filters (520) führen kann.

25. Gerät nach Anspruch 8, worin das expandierbare Drahtgeflecht oder Netz ferner ein Drahtgeflecht oder Netz aus Nitinol umfasst.

26. Ausrüstungssatz für den endovaskulären Ersatz einer erkrankten Herzklappe eines Patienten, wobei der Ausrüstungssatz Folgendes umfasst:
einen Valvuloplastik-Ballonkatheter (702); und
ein Gerät, das aufweist:
eine expandierbare Ersatzklappe (20) zur endovaskulären Abgabe und zum Einsetzen über der erkrankten Klappe des Patienten; und
ein Embolieschutzsystem (500) mit einer Außenhülle (510) und einem Emboliefilter (520), worin der Emboliefilter (520) zur endovaskulären Abgabe und zum Einsetzen stromabwärts von der kranken Klappe des Patienten konfiguriert ist, **dadurch gekennzeichnet, dass**
der Emboliefilter (520) aus einer kollabierten Abgabekonfiguration in eine expandierte eingesetzte Konfiguration expandiert werden kann, worin der Emboliefilter (520) zur Abgabe und/oder zur Rückholung im Lumen (512) der Hülle (510) kollabiert werden kann, worin das proximale Ende des Filters (520) zumindest teilweise von der Hülle (510) gelöst ist, wodurch während der Valvuloplastik oder während des Einsetzens der Ersatzklappe erzeugte Emboli umgelenkt werden können.

27. Ausrüstungssatz nach Anspruch 26, der ferner ein Abgabesystem zur endovaskulären Abgabe und zum Einsetzen der expandierbaren Ersatzklappe (20) umfasst.

28. Ausrüstungssatz nach Anspruch 26, der ferner ein Abgabesystem zur endovaskulären Abgabe und zum Einsetzen des Emboliefilters (520) umfasst.

## Revendications

1. Appareil de protection contre une embolisation au cours d'un remplacement endovasculaire de la valvule cardiaque d'un patient, l'appareil comportant :
une valvule (20) de remplacement configurée pour une administration et un déploiement endovasculaires ; et
un système (500) de protection embolique comprenant une gaine (510) extérieure et un filtre (520) embolique, dans lequel le filtre (520) embolique est configuré pour être placé en aval de la valvule (20) de remplacement au cours du déploiement de la valvule,
**caractérisé en ce que**
le filtre (520) embolique est configuré pour une expansion d'une configuration d'administration repliée à une configuration déployée d'expansion, dans lequel le filtre (520) embolique est configuré pour être replié afin d'être administré dans la lumière (512) de la gaine (510) et/ou d'y être retiré, dans lequel l'extrémité proximale du filtre (520) est au moins détachée partiellement de la gaine (510), redirigeant ainsi les emboles hors du système vasculaire cérébral du patient sans capturer les emboles dans le filtre (520).

2. Appareil selon la revendication 1, dans lequel le filtre (520) embolique est accouplé à la valvule (20) de remplacement.

3. Appareil selon la revendication 1, dans lequel le filtre (520) embolique est découplé de la valvule (20) de remplacement.

4. Appareil selon la revendication 1, dans lequel le filtre (520) embolique est configuré pour venir en contact avec l'aorte d'un patient et former un joint d'étanchéité circonférentiel contre l'aorte dans la configuration déployée.

5. Appareil selon la revendication 1, dans lequel le filtre (520) embolique est configuré pour une administration endovasculaire dans la configuration d'administration repliée.

6. Appareil selon la revendication 1, dans lequel la valvule (20) de remplacement est configurée pour une administration endovasculaire au travers du filtre (520) embolique.

7. Appareil selon la revendication 1 comportant en outre un élément de succion configuré pour aspirer les emboles redirigés hors de la circulation sanguine du patient.

8. Appareil selon la revendication 1, dans lequel le filtre (520) embolique est fabriqué à partir d'un fil tressé ou treillis métallique expansible.

9. Appareil selon la revendication 1, dans lequel le filtre (520) embolique comporte une structure enroulée en forme de spirale.

10. Appareil selon la revendication 9, dans lequel la structure enroulée en forme de spirale est configurée pour se dilater lorsqu'elle est serrée dans une première direction et se contracter lorsqu'elle est serrée dans une direction opposée.

11. Appareil selon la revendication 1, dans lequel le filtre (520) embolique comporte une membrane perméable d'une porosité spécifiée.

12. Appareil selon la revendication 11, dans lequel la porosité spécifiée comporte des pores inférieurs à environ 100 µm de diamètre.

13. Appareil selon la revendication 11, dans lequel la membrane perméable comporte une porosité variable.

14. Appareil selon la revendication 1 comportant en outre un ballonnet (702) expansible pour réaliser une valvuloplastie, dans lequel le filtre (520) embolique est configuré pour rediriger les emboles générés au cours de la valvuloplastie.

15. Appareil selon la revendication 1, dans lequel le filtre (520) embolique comporte au moins un élément de mesure pour déterminer les distances à l'intérieur du patient.

16. Appareil selon la revendication 16, dans lequel l'élément de mesure est configuré pour fournir une distance centre-axe entre la valvule cardiaque du patient et un emplacement souhaité à l'intérieur de l'aorte du patient.

17. Appareil selon la revendication 1, dans lequel le filtre (520) embolique comporte un profil courbe dans la configuration déployée.

18. Appareil selon la revendication 1, dans lequel le filtre (520) embolique est configuré pour se replier de la configuration déployée d'expansion à une configuration de retrait repliée.

19. Appareil selon la revendication 18 comportant en outre un élément de repliage conçu pour faciliter le repliage et le retrait du filtre (520) du patient.

20. Appareil selon la revendication 19, dans lequel le filtre (520) embolique comporte l'élément de repliage.

21. Appareil selon la revendication 20, dans lequel l'élément de repliage comporte une ouverture conique disposée au niveau d'une extrémité proximale du filtre (520) embolique.

22. Appareil selon la revendication 19, dans lequel l'élément de repliage comporte une gaine de retrait pouvant être avancée sur le filtre (520).

23. Appareil selon la revendication 4, dans lequel le filtre (520) embolique comporte en outre des interfaces (521) proximale et distale, et dans lequel le filtre embolique est configuré pour venir en contact avec l'aorte du patient seulement le long des interfaces (521) proximale et distale.

24. Appareil selon la revendication 6, dans lequel le filtre (520) embolique est configuré pour guider un cathéter d'une extrémité proximale du filtre (520) à une extrémité distale du filtre (520).

25. Appareil selon la revendication 8, dans lequel le fil tressé ou treillis métallique expansible comporte en outre un fil tressé ou un treillis métallique en nitinol.

26. Kit destiné au remplacement endovasculaire d'une valvule cardiaque malade d'un patient, le kit comportant :
un cathéter (702) à ballonnet pour valvuloplastie ; et
un appareil comportant :
une valvule (20) de remplacement expansible configurée pour une administration et un déploiement endovasculaire au travers de la valvule malade du patient ; et
un système (500) de protection embolique comprenant une gaine (510) extérieure et un filtre (520) embolique, dans lequel le filtre (520) embolique est configuré pour une administration et un déploiement endovasculaires en aval de la valvule malade du patient,
**caractérisé en ce que**
le filtre (520) embolique est configuré pour une expansion d'une configuration d'administration repliée à une configuration déployée d'expansion, dans lequel le filtre (520) embolique est configuré pour être replié afin d'être administré dans la lumière (512) de la gaine (510) et/ou d'y être retiré, dans lequel l'extrémité proximale du filtre (520) est au moins partiellement détachée de la gaine (510), redirigeant ainsi les emboles générés au cours d'une valvuloplastie ou du déploiement de la valvule de remplacement.

27. Kit selon la revendication 26 comportant en outre un système d'administration pour administrer et déployer par voie endovasculaire la valvule (20) de remplacement expansible.

28. Kit selon la revendication 26 comportant en outre un système d'administration pour administrer et déployer par voie endovasculaire le filtre (520) embolique.
